# EUROPÄISCHE PATENTANMELDUNG

(11) **EP 4 162 963 A1**
(43) Veröffentlichungstag der Anmeldung: **12.04.2023**
(21) Anmeldenummer: 21200856.9
(22) Anmeldetag: 05.10.2021
(51) Int. Cl.: A61M 5/24, A61M 5/315

(54) **VERABREICHUNGSVORRICHTUNG MIT VERBESSERTER BENUTZERFÜHRUNG**

(71) Anmelder: Ypsomed AG, 3401 Burgdorf (CH)
(72) Erfinder: Brügger, Martin, 3065 Bolligen (CH); Hostettler, Patrick, 3415 Hasle (CH)
(74) Vertreter: Meier Obertüfer, Jürg

(57) **Zusammenfassung**

Die Erfindung betrifft eine Verabreichungsvorrichtung (1) und ein Verfahren für die geführte Bedienung einer Verabreichungsvorrichtung (1) für ein Medikament (16) aufweisend: ein Vorwahlmittel (5) mit welchem eine Vorwahlmenge mechanisch oder elektronisch eingestellt und/oder gespeichert werden kann, einen manuell bewirkbaren oder freigebbaren motorischen Antrieb (10) welcher ein Abtriebsmittel (11) solange relativ zu einem in die Verabreichungsvorrichtung (1) einsetzbaren Behälter (15) enthaltend ein Medikament (16) bewegt, bis eine der Bewegung des Abtriebsmittels (11) entsprechende, aus dem Behälter (15) durch eine Nadel (13) und/oder Hohlkanüle (14) ausgeschüttete Ausschüttmenge die mit dem Vorwahlmittel (5) eingestellte oder gespeicherte Vorwahlmenge erreicht, ein manuell betätigbares Bedienmittel (4) welches einen unbetätigten und einen betätigten Zustand aufweisen kann und welches den Antrieb (10) im betätigten Zustand bewirken und/oder freigeben kann, eine elektronische Steuerung (6) enthaltend eine Zeitmesseinrichtung (7), wobei die Zeitmesseinrichtung (7) dazu ausgebildet ist, bei betätigtem Bedienmittel (4) eine Zeitdauer von dem Zeitpunkt ab welchem das Abtriebsmittel (11) vom Antrieb (10) nicht mehr bewegt wird bis zum Zeitpunkt in welchem das Bedienmittel (4) in den unbetätigten Zustand übergeht laufend zu messen.

## Beschreibung

### TECHNISCHES GEBIET

Die vorliegende Erfindung betrifft das Gebiet der Verabreichungsgeräte und Verabreichungssysteme für welche das Erzeugen, Auswerten, Speichern und Kommunizieren von Daten relevant ist. In Ausführungsformen bzw. Weiterbildungen bezieht sich die Erfindung auf Vorrichtungen, Systeme und Verfahren für die zuverlässige und genaue Erfassung und Auswertung von Daten im Zusammenhang mit der benutzungsfreundlichen und sicheren Dosierung und Verabreichung von Medikamenten im Rahmen einer Therapie, insbesondere einer Therapie über einen längeren oder unbestimmt langen Zeitraum.

### HINTERGRUND DER ERFINDUNG

Ausschüttungen mit Verabreichungsgeräten und/oder Verabreichungssystemen werden als therapierelevante Verabreichungsausschüttung, kurz Verabreichung bezeichnet, wenn sie im Rahmen einer Therapie in den Körper, beispielsweise das subkutane Gewebe eines Patienten erfolgen. Um eine genaue Dosierung zu gewährleisten, die Verabreichung von Luft anstelle von Medikament auszuschliessen und um die sichere Funktion des Geräts zu prüfen sind regelmässig auch Ausschüttungen auszuführen, bei denen die Nadel ausserhalb des Patientenkörpers bleibt, bzw. nicht eingestochen wird. Solche Ausschüttungen, bei denen das Geschehen an der Nadelspitze durch die Benutzerin beobachtbar ist, werden als Primingausschüttung, kurz Priming oder auch als "air shot" bezeichnet. Das so ausgeschüttete Medikament wird (als Primingmenge) entsprechend an die Umgebung abgegeben oder in ein dafür vorgesehenes Behältnis gelenkt und ist nicht therapierelevant. Dagegen ist bei einer therapierelevanten Verabreichung die eine Hohlkanüle aufweisende Nadel vor der Ausschüttung in beispielsweise das subkutane Gewebe einer Patientin einzustechen. Erst nach der Ausschüttung (einer Verabreichungsmenge) und nach einer vorgegebenen Verweildauer soll die Nadel wieder aus der Einstichstelle entfernt werden. Diese vorgegebene Verweildauer oder "dwell time" erlaubt insbesondere eine genügende Diffusion bzw. Verteilung des Medikaments in der vorgesehenen Dosis im Gewebe der Patientin. Der Therapieplan oder die Geräteanleitung bzw. ein Beipackzettel zum Medikament kann für die Verweildauer eine vorgeschriebene Dauer oder Mindestdauer vorgeben, beispielsweise 10 Sekunden nach dem Ende einer Auschüttung. Dabei kann es für eine Benutzerin oder einen Patienten schwierig sein, das Ende der Ausschüttung festzustellen und folglich den Beginn als auch die Dauer der vorgegebenen Verweildauer richtig zu bemessen.

Benutzer bzw. Patientinnen welche solche Mehrfach-Injektionsgeräte wie beispielsweise Insulinpens verwenden befolgen meist einen Verabreichungs- oder Therapieplan. Ein solcher kann im Tagesverlauf zu bestimmten Zeitpunkten und/oder Gelegenheiten eine Anzahl Verabreichungen mit je unterschiedlicher Dosis vorsehen. Benutzerinnen bzw. Patienten sind gehalten über alle Verabreichungen genau buchzuführen, insbesondere über die therapierelevanten Verabreichungsausschüttungen. Dies ist mühsam und anfällig für menschliche Fehlleistungen.

Aus dem Stand der Technik sind Offenbarungen bekannt, welche Vorrichtungen oder Verfahren für ein Priming von Verabreichungsgeräten bzw. ein Auseinanderhalten von Priming und Verabreichung lehren.

So offenbart die WO15066522A1 eine beispielhafte Ausführungsform die ein Benutzer vor einer Injektion vorbereiten kann. Der Benutzer beginnt die Injektionsvorrichtung zu entlüften, indem er die Injektionsvorrichtung und damit die Austrittsöffnung des Flüssigkeitsbehälters nach oben ausrichtet. Die Drehung der Injektionsvorrichtung in eine andere Ausrichtung kann von einem Sensor, wie z. B. einem Beschleunigungsmesser oder einem anderen Rotationssensor innerhalb der Injektionsvorrichtung, erfasst werden. Diese Drehung der Flüssigkeitsaustrittsöffnung nach oben kann es ermöglichen, dass Luft zur Oberseite der Vorrichtung und einer eventuell vorhandenen Nadel aufsteigt. Daher ist die Erfassung der Drehung der Flüssigkeitsaustrittsöffnung in die vorbestimmte Ausrichtung, in der die Flüssigkeitsaustrittsöffnung nach oben zeigt, in den Aspekten der gezeigten Lehre enthalten. Sobald die Flüssigkeitsaustrittsöffnung nach oben gedreht ist, kann der Benutzer auf ein Medikamentenreservoir tippen, um zu bewirken, dass verbliebene Luftblasen aufsteigen. In einer beispielhaften Ausführungsform kann ein mobiles Gerät eine Anweisung an einen Benutzer anzeigen, auf ein Medikamentenreservoir zu tippen. Sobald erkannt wurde, dass die Injektionsvorrichtung mit der Austrittsöffnung des Flüssigkeitsbehälters nach oben ausgerichtet ist, kann das Injektionssystem eine Eingabe, wie z. B. eine Priming-Taste oder einen Trigger, aktivieren. Der Benutzer führt dann eine Entlüftungsfunktion aus, indem er beispielsweise einen Touchscreen auf einem mit der Injektionsvorrichtung verbundenen mobilen Gerät verwendet, indem er die Entlüftungstaste oder den Auslöser auswählt oder aktiviert. Die Entlüftungsfunktion bewirkt dann, dass die Injektionsvorrichtung entlüftet wird, wobei die Luft durch die Flüssigkeit aus der Austrittsöffnung des Flüssigkeitsbehälters gedrückt wird.

Die WO1117404A2 offenbart eine Anzeige welche dazu verwendet werden kann, Informationen über das Priming der Medikamentenverabreichung zu liefern oder möglicherweise die Ausrichtung und/oder relative Position des Geräts anzuzeigen. Beispielsweise ist ein mikroelektromechanischer Beschleunigungsmesser in der Vorrichtung vorgesehen, so dass die Vorrichtung über die Intelligenz verfügt, zu erkennen, ob der Benutzer die Vorrichtung zur Durchführung eines Sicherheits- oder Priming-Ausschüttung verwendet (d. h., das distale Ende der Vorrichtung zeigt nach oben) oder ob er die Vorrichtung zur Durchführung eines Dosisverabreichungsschritts verwendet (d. h., das distale Ende der Vorrichtung zeigt nach unten). Das Display kann auch als Tagebuch oder Lebensstilkalender verwendet werden und vielleicht mit dem Glukosemessgerät des Patienten kommunizieren und vielleicht Glukosedaten speichern und anzeigen. Auf dem Display könnte auch eine Verweildauer nach der Abgabe einer Dosis angezeigt werden, die möglicherweise proportional zur Dosisgröße ist. Das Display könnte anzeigen, ob das Gerät aktiviert ist, d. h. bereit, eine Dosis abzugeben, und es könnte auch einen Hinweis geben, wenn die Dosis außerhalb der erwarteten Grenzwerte liegt.

In einer Ausführungsform der Publikation WO12160157A1 kann das Steuergerät so betrieben werden, dass es das Primingvolumen oder das Profil der intermittierenden Dosis auf einer Zufallsbasis festlegt. Dies kann unter Verwendung eines "Zufallskeims" geschehen, d. h. einer von einem Zufallszahlengenerator erzeugten Zufallszahl, die bei der Berechnung der Größe oder des Volumens einer oder mehrerer intermittierender Dosen verwendet wird. Das Primingvolumen kann aus einem Dosiermenü ausgewählt werden, optional in Abhängigkeit von der Verweildauer (d.h. wie lange die Dosiertaste gedrückt wird) der Benutzeraktivierung der Benutzeroberfläche. Im Primingmenü kann eine grundlegende Vorkonfiguration eingestellt werden. Diese Optionen können von Vorteil sein, wenn Nadeln unterschiedlicher Größe für die Verwendung mit dem Gerät in Betracht gezogen werden. Weiter wird ein Verfahren zum Steuern eines handgehaltenen Insulininjektionspens zum Ausschütten eines oder mehrerer Arzneimittel gezeigt, wobei der Pen mindestens einen Primingmodus und einen Arzneimittelabgabemodus zum Verabreichen des einen oder der mehreren Arzneimittel aufweist, wobei das Verfahren umfasst: Steuern des Pens um einen Initialisierungsvorgang durchzuführen; und Verabreichen einer Vielzahl von intermittierenden Dosen eines Medikaments in Abhängigkeit von der Aktivierung des Primingvorgangs durch eine Benutzerschnittstelle, wobei die Vielzahl von intermittierenden Dosen eine vorbestimmte Sequenz von Primingdosen umfasst.

Die Offenbarung in der Publikation WO2021/041378A1 bezieht sich auf Verfahren zur Bestimmung von Dosisinformationen von Injektionen. Dabei wird auf Daten einer Injektionsereignisgruppe zugegriffen, welche ein oder mehrere Injektionsereignisse auf einem oder mehreren Geräten betreffen. Die Injektionsereignisgruppe wird verarbeitet um eine tatsächlich verabreichte Dosis eines bestimmten Injektionsereignisses basierend auf einem oder mehrerer der folgenden Kriterien zu bestimmen: wie das Injektionsereignis mit einem Gerätewechsel zusammenhängt; wie das Injektionsereignis bezüglich der Dosisgrösse mit anderen Ereignissen in der Gruppe einzuordnen ist; welche Position es in der Injektionsereignisgruppe einnimmt und/oder mit welcher Zeitdauer es zu benachbarten Injektionen der Injektionsereignisgruppe beabstandet ist.

Die WO2021/116052A1 offenbart eine Verabreichungsvorrichtung für Medikamente mit einem Dosisabgabeknopf und einem "end of dose" oder "0U" Schalter oder Sensor welcher geschlossen bzw. aktiviert wird, kurz bevor eine laufende Ausschüttung beendet ist, d.h.. bevor ein Anschlag die laufende Ausschüttung mechanisch beendet. Dabei ergibt sich immer die Möglichkeit, dass der Benutzer den Dosisabgabeknopf zu früh loslässt, und damit eine Ausschüttung zu früh abbricht. Deshalb ist eine Verzögerungszeit vorgesehen, welche diese Problematik mildert. Dies erfolgt indem die Verzögerungszeit an der menschlichen Reaktionszeit (oder wenig darunter) bemessen wird. Somit wird grossmehrheitlich sichergestellt, dass die Dosen als voll ausgeschüttet aufgezeichnet werden, bevor ein Benutzer auf ein "end of dose" Signal reagieren und den Dosisabgabeknopf loslassen kann. In anderen Fällen wo der Benutzer den Dosisabgabeknopf bei laufender Ausschüttung loslässt, wird die Dosis als unvollständig ausgeschüttet aufgezeichnet.

Für eine sichere, ungefährliche Dosierung oder Therapie ist es wichtig, die wirksam verabreichte Menge eines Medikaments zu kennen. Ausschüttungen welche bloss dem Entlüften dienen dürfen nicht als verabreichte Menge gezählt und registriert werden. Die im oben zitierten Stand der Technik gezeigten Lösungen mit beispielsweise Lagesensoren oder Gyroskopen sind unsicher, da das Gerät für einen Entlüftungsvorgang aus der vorgeschrieben Lage im Raum gekippt oder geschwenkt werden kann. Ebenso kann nicht ausgeschlossen werden, dass eine wirksame Menge in einer solchen Lage verabreicht wird. Eine Entlüftung nur in einem speziell dazu vorgesehenen Betriebsmodus zu ermöglichen ist umständlich und birgt die Gefahr einer Fehlanwendung. Eine Entlüftung anhand einer kleinen Menge zu erkennen schränkt die Möglichkeiten einer effizienten Entlüftung als auch eines weiten Verabreichungsbereichs ein. Verfahren, welche auf bestimmten vordefinierten Mustern beruhen sind nur für vorbestimmte Fälle anwendbar und sind anfällig für menschliche Ungenauigkeiten.

Solche aus dem Stand der Technik bekannten oder bestimmbaren Parameter von Verfahren und Vorrichtungen beziehen sich auf bestimmte vordefinierte Bedingungen oder vordefinierte Kriterien, d.h. auf ideale bzw. vorhersehbare Verabreichungsumgebungen und/oder Verabreichungsszenarien oder auf bestimmte vordefinierte Startwerte und sind deshalb oft nicht oder nur ungenügend geeignet oder nicht sicher anwendbar auf ein instantanes, d.h. unmittelbar sich ergebendes Verabreichungsszenario oder auf ein unmittelbar sich ergebenes Benutzerbedürfnis- oder Therapieerfordernis .

### DARSTELLUNG DER ERFINDUNG

Es ist eine zugrundeliegende Aufgabe der vorliegenden Erfindung eine Verabreichungsvorrichtung zur parenteralen Verabreichung eines Medikaments sowie Verfahren bereitzustellen, welche die sichere Benutzung der Vorrichtung vereinfachen und dadurch die Sicherheit in der Anwendung zu erhöhen. Es ist weiter eine Aufgabe der vorliegenden Erfindung Vorrichtungen und Verfahren bereitzustellen, welche sicher anwendbar sind auf ein unmittelbar sich ergebendes Verabreichungsszenario oder auf ein instantanes Benutzerbedürfnis oder Therapieerfordernis.

Die Aufgabe wird gelöst durch Vorrichtungen und Verfahren gemäss den unabhängigen Ansprüchen. Vorteilhafte Weiterbildungen gehen aus der Erfindungsdarstellung und/oder den abhängigen Ansprüchen hervor. Die dargestellten vorteilhaften Weiterbildungen und Aspekte sind weitgehend unabhängig voneinander oder beliebig kombinierbar.

In einem Aspekt der vorliegenden Erfindung wird eine verbesserte Unterscheidung von Priming und Verabreichung dargestellt. Weitere Aspekte der Erfindung betreffen ihre Anwendung im Rahmen von technischen Systemen und Verfahren welche im Rahmen von medikamentösen Therapien zur Anwendung gelangen, beispielsweise in diabetologischen Systemen, welche neben den Verabreichungsvorrichtungen weiter Glukosemessgeräte, insbesondere zur kontinuierlichen Glukosemessung, Fernsteuerungen, Bolusrechner sowie Hostsysteme aufweisen können. Die Aspekte der vorliegenden Erfindung unterstützen den Benutzer bei der fehlerfreien und einfachen Handhabung solcher Systeme was zu verbesserten Therapieresultaten führt.

Weiter kann die vorliegende Erfindung die Genauigkeit einer Berechnung des "insulin on board" IOB verbessern, indem sie die Qualität der Eingabegrössen für die IOB Berechnung optimiert. Der berechnete IOB Wert macht eine Aussage über das aktive Insulin im Körper des Patienten zu einem bestimmten Zeitpunkt, wobei die Genauigkeit der Berechnung auch von der Qualität der erfindungsgemäss verbesserten Aufzeichnung der Verabreichungsgeschichte profitiert, indem die vorliegende Erfindung die Registrierung der Zeitpunkte und Verabreichungsmengen verbessert und automatisiert. Letztlich führt ein genauer IOB Wert zu einem genaueren Bolusvorschlag und damit zu einer verbesserten Therapie.

Beispielsweise kann die vorliegende Erfindung die "time in range" TIR, d.h. die Zeitdauer im glykämischen Zielbereich verbessern. Mit der zunehmenden Verbreitung der Diabetestechnologie führt die TIR als Alternative und/oder Ergänzung zum HbAlc zu einem Paradigmenwechsel in der Diabetologie. Durch die kontinuierliche Glukosemessung ist es möglich geworden, Muster zu identifizieren ― in Bezug auf steigende/fallende Werte zu bestimmten Tageszeiten oder nach bestimmten Mahlzeiten. Diese lassen sich zur Anpassung der Therapie nutzen. Die "time in range" (TIR, Zeit im Zielbereich) ist Indikator für die Dauer der normo-, hypo- und hyperglykämischen Phasen. In Analogie zur Bewertung der Blutdruckeinstellung kann dieser Parameter durch 2 Werte ausgedrückt werden:
- die prozentuale Zeit im Zielbereich 70-180 mg/dl (3,9-10,0 mmol/L) und
- die Zeit im hypoglykämischen Bereich< 70 mg/dl/< 3,9 mmol/L

### Beispiele für TIR Parameter: 52/15 % oder 84 / 0 %

Weiter kann die vorliegende Erfindung in einem ihrer Aspekte die Bedienung einer Verabreichungsvorrichtung durch eine intuitive Benutzerführung vereinfachen indem korrekte Dosiseinstellungen und richtige Verabreichungszeitpunkte direkt auf der Verabreichungsvorrichtung qualitativ signalisierbar sind. Dadurch wird vor und während der Verabreichung eine einfache, eindeutige Bedienhilfe angeboten und ein quantitativ (numerisches) Einstellen von Dosiswerten wird fakultativ oder entfällt ganz.

Die vorliegende Erfindung betrifft eine Verabreichungsvorrichtung zur parenteralen Verabreichung eines Medikaments aufweisend,
ein Vorwahlmittel mit welchem eine Vorwahlmenge mechanisch oder elektronisch eingestellt und/oder gespeichert werden kann,
einen manuell bewirkbaren Antrieb oder einen freigebbaren motorischen Antrieb welcher ein Abtriebsmittel (Kolbenstange, Gewindestange, Segmentstange, Flansch, Balg, Kolben, Rolle etc.) solange relativ zu einem in die Verabreichungsvorrichtung einsetzbaren Behälter (Karpule, Spritze, Beutel etc.) enthaltend ein Medikament bewegt, bis eine der Bewegung des Abtriebsmittels entsprechende, aus dem Behälter (15) durch eine Nadel (13) und/oder Hohlkanüle (14) ausgeschüttete Ausschüttmenge die mit dem Vorwahlmittel (5) eingestellte oder gespeicherte Vorwahlmenge erreicht oder ein anderes Stopkriterium eintritt,
ein manuell betätigbares Bedienmittel (Druckknopf, Schieber, Taster, Schalter etc.) welches einen unbetätigten und einen betätigten Zustand aufweisen kann und welches den Antrieb im betätigten Zustand bewirken und/oder freigeben kann,
eine elektronische Steuerung enthaltend eine Zeitmesseinrichtung,
wobei die Zeitmesseinrichtung dazu ausgebildet ist, bei betätigtem Bedienmittel eine Zeitdauer von dem Zeitpunkt ab welchem das Abtriebsmittel vom Antrieb nicht mehr bewegt wird bis zum Zeitpunkt in welchem das Bedienmittel in den unbetätigten Zustand übergeht laufend zu messen, insbesondere längstens bis zum Zeitpunkt in welchem das Bedienmittel in den unbetätigten Zustand übergeht laufend zu messen.

Die derart laufend ermittelte und schliesslich erreichte Zeitdauer bildet Ausgangspunkt für Weiterbildungen der vorliegenden Erfindung. Die schliesslich erreichte Zeitdauer kann auch als Haltedauer bezeichnet werden und ist im Wesentlichen durch die Benutzerin oder den Patienten über die Betätigung des Bediensmittels bestimmbar, wodurch bewusst oder intuitiv auf eine laufende und eine nachfolgende Auswertung, Charakterisierung bzw. die Zweckbestimmung einer Ausschüttung Einfluss genommen werden kann bzw. wird.

Ein anderes Stopkriterum kann beispielsweise ein mechanischer Null-Anschlag, ein Endschalter, ein leerer Behälter oder ein unbetätigtes Bedienmittel (d.h. wenn das Bedienmittel bei laufendem Antrieb in den unbetätigten Zustand wechselt) oder ein Gerätefehler beispielsweise ein Antriebsfehler oder ein Sensorikfehler oder eine erkannte Verstopfung oder Blockierung der Nadel oder des Ausschüttpfads sein bzw. liefern.

Vorteilhaft ist die Verabreichungsvorrichtung weitergebildet indem die elektronische Steuerung dazu ausgebildet ist, die Ausschüttmenge als Primingmenge zu charakterisieren solange bzw. wenn die Zeitdauer kleiner ist als eine vordefinierte Grenzdauer und/oder die Ausschüttmenge als Verabreichungsmenge zu charakterisieren sobald bzw. wenn die Zeitdauer gleich oder grösser ist als die vordefinierte Grenzdauer. Insbesondere kann die elektronische Steuerung jederzeit aktuell die Relation der Zeitdauer zu der vordefinierten Grenzdauer bestimmen um zu ermitteln, ob eine Auschüttung als Priming oder als Verabreichung zu charakterisieren ist. Das hat den Vorteil, dass ein Benutzer oder eine Patientin intuitiv durch die vorgesehene Handhabung der Verabreichungsvorrichtung angibt, ob eine einzelne Ausschüttung zwecks Priming oder als Verabreichung getätigt wurde.

Vorteilhaft weitergebildet indem die vordefinierte Grenzdauer als Wert aus einem Bereich von 1 bis 20 sec, bevorzugt 2 bis 5 sec, noch bevorzugter auf 3 sec in der elektronischen Steuerung festgelegt ist oder aus einem Kontroller (Fernsteuerung, Smartphone App, BGM, CGM, PC, Netzwerk, Cloud, Host etc.) veränderbar vorgegeben werden kann. Beispielsweise kann die vordefinierte Grenzdauer kleiner sein als eine auf dem Beipackzettel zum Medikament vorgegebene Verweildauer oder mit dieser übereinstimmen, wodurch eine Benutzerin bei der Bemessung der Verweildauer durch die Verabreichungsvorrichtung unterstützt bzw. geführt wird und hat den Vorteil, dass individuelle Patientenbedürfnisse oder spezielle medizinische, pharmazeutische oder physiologische Gegebenheiten betreffend eine Variation der Verweildauer berücksichtigt werden können ohne die Sicherheit der Unterscheidung von Priming und Verabreichung zu schmälern.

Vorteilhaft weitergebildet indem eine vorgegebene Verweildauer als Wert aus einem Bereich von 0 bis 20 sec, bevorzugt 5 bis 12 sec, noch bevorzugter auf 10 sec festgelegt ist oder aus einem Kontroller veränderbar vorgegeben werden kann. Die vorgegebene Verweildauer kann erfindungsgemäss auch kürzer oder länger bemessen sein, als eine gemäss bestimmungsgemässem Gebrauch festgelegte Verweildauer. Dies hat den Vorteil, dass individuelle Patientenbedürfnisse oder spezielle medizinische, pharmazeutische oder physiologische Gegebenheiten betreffend eine Variation der Verweildauer berücksichtigt werden können ohne die Sicherheit der Unterscheidung von Priming und Verabreichung zu schmälern.

Vorteilhaft weitergebildet indem die elektronische Steuerung den Stillstand des Abtriebsmittels aufgrund eines oder mehrere Antriebsparameter (beispielsweise Strom, GegenEMK, Ableitungen daraus) oder mittels einem Endschalter oder Sensor oder einem rotativen oder linearen Encoder feststellen kann.

Vorteilhaft weitergebildet indem die elektronische Steuerung einen Sensor, beispielsweise einen Encoder oder aufweist, mit welchem die der Bewegung des Abtriebsmittels entsprechende Menge an Medikament oder die Auschüttmenge quantitativ ermittelt werden kann.

Vorteilhaft weitergebildet indem der Behälter eine Karpule mit Stopfen und Septum ist. Beispielsweise können solche Karpulen ein Nutzvolumen von 3,0 ml oder 1,6 ml aufweisen.

Vorteilhaft weitergebildet indem die elektronische Steuerung eine Echtzeituhr aufweist, wodurch Ereignisse oder Statusänderungen, insbesondere Ausschüttmengen mit einem Zeit- und/oder Datumsstempel ergänzbar sind.

Vorteilhaft weitergebildet indem die Echtzeituhr mit einer Uhr auf dem Kontroller synchronisierbar ist.

Vorteilhaft weitergebildet indem die elektronische Steuerung einen Temperatursensor aufweist, womit der Temperaturverlauf während Betrieb und Lagerung überwacht und wodurch Ereignisse oder Statusänderungen mit einer Temperaturangabe ergänzbar sind. Dies erlaubt auch die Sicherstellung der Medikamentenqualität durch Feststellung der Temperaturminima und -Maxima denen ein eingesetzter Behälter oder das darin enthaltene Medikament ausgesetzt ist bzw. war. Der Temperatursensor ist vorteilhaft in der Nähe des Behälters angebracht und/oder mit diesem thermisch gekoppelt.

Vorteilhaft weitergebildet indem die elektronische Steuerung einen Speicher aufweist, welcher zumindest die letzte der Bewegung des Abtriebsmittels entsprechende Ausschüttmenge als Verabreichungsmenge aufzeichnet, sofern diese nicht als Primingmenge charakterisiert ist. Wobei die Ausschüttmenge bzw. die Verabreichungsmenge und/oder die Primingmenge je mit einem Zeitstempel und/oder Datumsstempel und/oder zumindest einer weiteren Charakterisierung (Medikament, Konzentration, Lotnummer, Karpulennummer, Temperatur, geografischer Ort, räumliche Orientierung., Konformität mit dem bestimmungsgemässen Gebrauch etc.) versehen sein können.

Vorteilhaft weitergebildet indem die elektronische Steuerung weitere Speicher für die Summe von mehreren Verabreichungsmengen und/oder Primingmengen aufweist.

Vorteilhaft weitergebildet indem die elektronische Steuerung weitere Speicher für je eine Vielzahl aufeinanderfolgender Verabreichungsmengen und/oder Primingmengen aufweist, wobei die Vielzahl der aufeinanderfolgenden Verabreichungsmengen und/oder Primingmengen je mit einem Zeitstempel und/oder einer Charakterisierung (Medikament, Konzentration, Lotnummer, Karpulennummer, Temperatur, geografischer Ort, räumliche Orientierung etc.) versehen sein können. Dadurch wird eine verbesserte Aufzeichnung der Verabreichungsgeschichte möglich.

Vorteilhaft weitergebildet indem die elektronische Steuerung einen Sendeempfänger aufweisen kann um zu einem Kontroller eine drahtlose Verbindung herzustellen, realisiert beispielsweise durch Bluetooth Low Energy BLE, Near Field Communication NFC, Ultra Wideband UWB Technologie, wobei eine Sicherung durch geeignete Authentifizierung und Verschlüsselung angewendet werden kann.

Vorteilhaft weitergebildet indem der Kontroller eines aus beispielsweise Fernsteuerung, Smartphone App, BGM, CGM, PC, Netzwerk, Cloud, Host etc. sein kann oder der Kontroller kann ein System von mehreren aus Fernsteuerung, Smartphone App, BGM, CGM, PC, Netzwerk, Cloud, Host etc. sein. Insbesondere kann der Kontroller im Normalbetrieb auch bloss ein CGM Gerät sein, wodurch auf die Interaktion beispielsweise mit einer Smartphone App gänzlich verzichtet werden kann oder eine solche nur bei einer Inbetriebnahme eines neuen CGM Geräts oder Glukosesensors notwendig ist. Dies erlaubt der Benutzerin oder dem Patienten eine einfachere Anwendung auch in Situationen wo beispielsweise ein Smartphone nicht zur Verfügung stehen kann. Alternativ kann die Funktionalität des Kontrollers auch in der elektronischen Steuerung in der Verabreichungsvorrichtung selber implementiert sein. Dies erlaubt beispielsweise die Kommunikation/Interaktion mit einem kontinuierlichen Blutglukosemessgerät CGM ohne weitere Systemkomponenten. Zudem ist dadurch die erfindungsgemässe Verabreichungsvorrichtung auch temporär oder dauernd autark betreibbar.

Vorteilhaft weitergebildet indem die elektronische Steuerung über die insbesondere gesicherte drahtlose Verbindung vom Kontroller einen Vorgabewert für eine nächste Ausschüttung empfangen kann, zusätzlich kann angegeben sein, ob der Vorgabewert eine Verabreichung oder ein Priming betrifft. Ein solcher Vorgabewert ist beispielsweise durch einen im Kontroller implementierten Bolusrechner aufgrund von Eingaben und der Verabreichungsgeschichte sowie individueller Parameter berechenbar. Beispielsweise kann auf einer Smartphone App je ein Wert für den aktuell gemessenen Glukosespiegel und den Energiegehalt der unmittelbar bevorstehenden Mahlzeit eingegeben werden. Aus diesen Eingaben, dem IOB und weiteren individuellen Parametern berechnet der Bolusrechner eine Insulindosis, welche durch die Benutzerin oder den Patienten durch eine Eingabe bestätigt als Vorgabewert an die Verabreichungsvorrichtung sendbar ist. Ein solcher Bolusrechner oder der Kontroller ist auch in der Lage aufgrund beispielsweise der aktuellen CGM Daten spontan Korrekturboli vorzuschlagen und/oder an die Verabreichungsvorrichtung zu übertragen.

Vorteilhaft weitergebildet indem die elektronische Steuerung über die gesicherte drahtlose Verbindung zum Kontroller die aktuellste gespeicherte Verabreichungsmenge und/oder Primingmenge übertragen kann und/oder die Vielzahl aufeinanderfolgender Verabreichungsmengen und/oder Primingmengen inklusive zugehörige Zeitstempel und Charakterisierungen übertragen kann.

Vorteilhaft weitergebildet indem die Verabreichungsvorrichtung eine Skala (bedruckte Hülse mit Zeiger, LCD, LED, ePaper etc.) aufweist, welche die aktuell mit der Vorwahlmittel vorgewählte Menge quantitativ, insbesondere als eine Anzahl Einheiten anzeigen kann.

Vorteilhaft weitergebildet indem die aktuell mit dem Vorwahlmittel vorgewählte Menge zusätzlich oder alternativ durch den Kontroller angezeigt werden kann, beispielsweise auf einem Bildschirm eines Smartphone, was die Ablesbarkeit und Bediensicherheit verbessert.

Vorteilhaft weitergebildet indem die Verabreichungsvorrichtung und/oder der Kontroller ein Anzeigemittel (mehrfarbige oder monochrome LED, LCD, ePaper, Sprachausgabe, Summer etc.) aufweist, welches von der elektronischen Steuerung angesteuert signalisieren kann, dass die aktuelle mit dem Vorwahlmittel eingestellte Vorwahlmenge zumindest eines aus kleiner, gleich, etwas grösser, viel grösser als der Vorgabewert ist und/oder die Auschüttmenge die mit dem Vorwahlmittel vorgewählte oder die gespeicherte Vorwahlmenge erreicht hat und /oder die aktuelle Zeitdauer zumindest eines aus kleiner, gleich, etwas grösser, viel grösser als die vordefinierte Grenzdauer und/oder eine vorgegebene Verweildauer ist und/oder ein Stopkriterium beispielsweise ein Fehler aufgetreten ist. Dadurch kann die Korrektheit der Vorwahlmenge und/oder die Charakterisierung bzw. die Therapierelevanz der Ausschüttung und/oder die Verweilweilzeit der Nadel im Gewebe gemäss bestimmungsgemässen Gebrauch qualitativ angezeigt werden, was die Benutzerführung erleichtert und die Sicherheit der Verabreichungsvorrichtung erhöht.

Voreilhaft weitergebildet indem bei einer Verweildauer der Nadel im Gewebe gemäss bestimmungsgemässen Gebrauch bzw. wenn die Zeitdauer grösser oder gleich der vorgegebenen Verweildauer ist, eine Verabreichung als konform charakterisiert werden kann. Insbesondere kann die gemessene Zeitdauer aktuell oder rückschauend herangezogen werden um zu ermitteln, ob eine Verabreichung mit dem bestimmungsgemässen Gebrauch konform ist.

Voreilhaft weitergebildet indem bei einer Zeitdauer kleiner der vorgegebenen Verweildauer die Verabreichungsmenge um die demzufolge nicht ins Gewebe diffundierte Menge an Medikament vermindert oder die Verabreichung bzw. Verabreichungsmenge anulliert werden kann.

Das Anzeigemittel weist beispielsweise eine oder mehrere mehrfarbige LED auf, welche bezüglich emittierter Lichtfarbe und/oder Lichtintensität und/oder bezüglich Impulsdauer bzw. Tastverhältnis moduliert ansteuerbar ist. Alternativ oder ergänzend kann das Anzeigemittel auch eine oder mehrere einfarbige LED aufweisen. Ein solches Anzeigemittel kann beispielsweise eine, insbesondere mehrere der folgenden Signalisierungen aufweisen:

**Bedienmittel (Knopf) unbetätigt:**

| Farbe/ Tastverhältnis | weiss | grün | rot |
|---|---|---|---|
| dauerhaft | Vorwahlmenge < > Vorgabewert | Vorwahlmenge = Vorgabewert | Vorwahlmenge >> Vorgabewert |
| blinkend | | | Gerätefehler |

**Bedienmittel (Knopf) betätigt:**

| Farbe/ Tastverhältnis | weiss | grün | rot |
|---|---|---|---|
| dauerhaft | | Zeitdauer >= Grenz-/Verweildauer (Verabreichung beendet) | |
| blinkend | Vorwahlmenge < > Vorgabewert, Zeitdauer < Grenz-/Verweildauer | Vorwahlmenge = Vorgabewert, Zeitdauer < Grenz-/Verweildauer | Vorwahlmenge >> Vorgabewert, Zeitdauer < Grenz-/Verweildauer |

Vorteilhaft weitergebildet indem das Anzeigemittel mit unterscheidbarem Tastverhältnis oder einer anderen Modulation signalisiert, ob die vordefinierte Grenzdauer nicht erreicht ist oder überschritten ist. So kann die Benutzerin erkennen, ob eine aktuelle Ausschüttung als Priming oder als Verabreichung charakterisiert ist.

Vorteilhaft weitergebildet indem das Anzeigemittel mit unterscheidbarem Tastverhältnis oder einer anderen Modulation signalisiert, ob die vorgegebene Verweildauer nicht erreicht ist oder überschritten ist. So kann der Benutzer erkennen, ob eine aktuelle Ausschüttung konform verabreicht ist, beispielsweise gemäss bestimmungsgemässen Gebrauch.

Vorteilhaft weitergebildet indem die Verabreichungsvorrichtung und/oder der Kontroller ein zweites Anzeigemittel aufweist, mit dem der Zustand der insbesondere gesicherten drahtlosen Verbindung signalisierbar ist, beispielsweise eine blaue LED.

Vorteilhaft weitergebildet indem die mit dem Vorwahlmittel einstellbare bzw. eingestellte Vorwahlmenge oder der gespeicherte Vorgabewert aus dem Kontroller verändert, festgelegt oder begrenzt werden kann.

Vorteilhaft weitergebildet ist der Vorgabewert aus einem in der Verabreichungsvorrichtung gespeicherten Plan oder Schema insbesondere zeitgesteuert entnehmbar, insbesondere zeitgesteuert und mit einer geeigneten Signalisierung über beispielsweise das Anzeigemittel und/oder akustische und/oder haptische Signalgeber und/oder durch Signalisierungsmittel und/oder Alarme im Kontroller, beispielsweise der Smartphone App. Dies erlaubt die Verabreichungsvorrichtung temporär oder dauerhaft autark zu betreiben.

Vorteilhaft weitergebildet indem die Verabreichungsvorrichtung periodisch und/oder spontan und/oder nach einer Ausschüttung und/oder auf Anfrage durch den Kontroller die Inhalte der Speicher und/oder den Status der Verabreichungsvorrichtung einzeln oder gesamthaft an den Kontroller überträgt.

Die Erfindung betrifft weiter ein Verfahren, insbesondere ein computergestütztes Verfahren für die geführte Bedienung einer Verabreichungsvorrichtung zur Verabreichung eines Medikaments aufweisend die Schritte:
- Bereitstellen eines Vorgabewerts und einer vordefinierten Grenzdauer auf einem Kontroller
- Übertragen des Vorgabewerts und der vordefinierten Grenzdauer vom Kontroller an die Verabreichungsvorrichtung oder die elektronische Steuerung
- Einstellen einer Vorwahlmenge an der Verabreichungsvorrichtung
- Signalisieren der Relation Vorwahlmenge zu Vorgabewert durch die Verabreichungsvorrichtung, insbesondere signalisieren, dass die Vorwahlmenge identisch zum Vorgabewert ist und/oder, dass die Verabreichungsvorrichtung bereit ist für eine Verabreichung
- Betätigen des Bedienmittels an der Verabreichungsvorrichtung wodurch Ausschütten von Medikament als Ausschüttung erfolgt
- Laufendes Messen einer Zeitdauer ab dem Ende der Ausschüttung bis zur Unbetätigung des Bedienmittels

Vorteilhaft weitergebildet ist das Verfahren für die geführte Bedienung einer Verabreichungsvorrichtung zur Verabreichung eines Medikaments indem es zudem einen oder mehrere der folgenden Schritte aufweist:
- Signalisieren, dass ein Vorgabewert empfangen oder verändert wurde, beispielsweise durch eine Anzeige und/oder einen akustischen Signalgeber
- Festhalten der Zeitdauer vom Ende der Ausschüttung bis zur Unbetätigung des Bedienmittels als Haltedauer
- Erstellen einer drahtlosen authentifizierten Paarung der Verabreichungsvorrichtung und dem Kontroller, insbesondere durch eine "out of band" Methode, beispielsweise mittels QR Code auf der Verabreichungsvorrichtung oder dem darin einsetzbaren Medikamentenbehälter
- Erstellen einer kryptographisch gesicherten drahtlosen Verbindung zwischen der Verabreichungsvorrichtung und dem Kontroller
- Abgleich der gespeicherten Auschüttmengen, Charakterisierungen, Vorgabewerte, Stati, Parameter, Echtzeituhren, geografischen Koordinaten zwischen der Verabreichungsvorrichtung und dem Kontroller
- Übertragen einer vorgegebenen Verweildauer vom Kontroller an die Verabreichungsvorrichtung oder die elektronische Steuerung
- Signalisieren, dass die aktuell eingestellte Vorwahlmenge kleiner als der Vorgabewert ist
- Signalisieren, dass die aktuell eingestellte Vorwahlmenge gleich dem Vorgabewert ist
- Signalisieren, dass die aktuell eingestellte Vorwahlmenge grösser als der Vorgabewert ist
- Signalisieren des Endes der Ausschüttung sobald die eingestellte Vorwahlmenge oder eine Ausschüttmenge ausgeschüttet ist
- Signalisieren, dass die Zeitdauer die vordefinierte Grenzdauer nicht erreicht
- Signalisieren, dass die Zeitdauer die vordefinierte Grenzdauer erreicht oder überschreitet

- Signalisieren, dass die Zeitdauer eine vorgegebene Verweildauer nicht erreicht
- Signalisieren, dass die Zeitdauer eine vorgegebene Verweildauer erreicht oder überschreitet
- Versehen der Ausschüttmenge mit einem oder mehreren aus Zeitstempel, Medikamentenbezeichnung, Konzentration, Lotnummer, Karpulennummer, Temperatur, geografischer Ort, räumliche Orientierung etc.
- Charkterisieren der Ausschüttmenge als Primingmenge falls die Zeitdauer kleiner als die vordefinierte Grenzdauer ist
- Charkterisieren der Ausschüttmenge als Verabreichungsmenge falls die Zeitdauer grösser oder gleich der vordefinierte Grenzdauer ist
- Charkterisieren der Ausschüttmenge als konforme Verabreichungsmenge falls die Zeitdauer grösser oder gleich der vorgegebenen Verweildauer ist
- Löschen des Vorgabewerts falls die Verabreichungsmenge gleich oder grösser ist als der Vorgabewert
- Speichern der Verabreichungsmenge in der Verabreichungsvorrichtung
- Speichern der Primingmenge in der Verabreichungsvorrichtung
- Übertragen der Verabreichungsmenge von der Verabreichungsvorrichtung an den Kontroller
- Übertragen der Primingmenge von der Verabreichungsvorrichtung an den Kontroller
- Abfragen einer Bestätigung auf dem Kontroller zur Verifikation der letzten Verabreichungsmenge und/oder Primingmenge

Für Benutzungsfälle bei denen die Verabreichungsmenge kleiner ist als der Vorgabewert ist das Verfahren für die geführte Bedienung einer Verabreichungsvorrichtung zur Verabreichung eines Medikaments vorteilhaft weitergebildet indem es zudem einen oder mehrere der folgenden Schritte aufweist:
- Vermindern des Vorgabewerts um die Verabreichungsmenge
- Übertragen des verminderten Vorgabewerts an den Kontroller
- Aufforderung auf dem Kontroller zum Wechsel des Behälters, insbesondere einer Karpule in der Verabreichungsvorrichtung
- Aufforderung auf dem Kontroller zum Durchführen von Priming an der Verabreichungsvorrichtung

Ein solcher Benutzungsfall kann sich ergeben, wenn die Restmenge an Medikament im Behälter nicht ausreicht um eine Vorwahlmenge bzw. Ausschüttmenge in der Höhe des Vorgabewerts zu tätigen. In diesem Fall ist ein Wechsel des Behälters bzw. der Karpule vorzunehmen. In einem anderen Benutzungsfall kann die Benutzerin oder der Patient einen Vorgabewert mittels zwei oder mehreren Verabreichungen erreichen - absichtlich oder weil eine erste Verabreichung durch einen Gerätefehler frühzeitig beendet wird.

Die Erfindung betrifft weiter das Verfahren, insbesondere das computergestütztes Verfahren für die geführte Bedienung einer Verabreichungsvorrichtung zur Verabreichung eines Medikaments ausgeführt auf einer Verabreichungsvorrichtung für eine Verabreichungsvorrichtung für ein Medikament und/oder ausgeführt auf einer Verabreichungsvorrichtung für ein Medikament.

Weitere Aspekte von bevorzugten Weiterbildungen und deren Ausführungsformen sind dokumentiert in der europäischen Anmeldung EP 21187086.0, deren Lehre in die vorliegende Anmeldung vollumfänglich durch Verweis aufgenommen wird (incorporation by reference).

Der Begriff "Verabreichungsgerät" umfasst Injektionsgeräte welche bestimmte Dosen von Medikament ausschütten können. Mehrfach-Injektionsgeräte enthalten meist einen Behälter für das Medikament und eine Dosiseinstelleinrichtung, welche der Benutzerin erlaubt, eine gewünschte Dosis für jede einzelne Ausschüttung vorzuwählen. Weiter sind Mehrfach-Injektionsgeräte mit einer eine Hohlkanüle aufweisenden Nadel ausgerüstet oder ausrüstbar, durch welche das Medikament ausschüttbar ist.

Insulinpens sind beispielsweise solche Mehrfach-Injektionsgeräte welche bekannterweise für die Behandlung von Diabetes mellitus zur Anwendung gelangen. Insulinpens enthalten eine Karpule mit einer insulinhaltigen Lösung, welche für eine Vielzahl von Ausschüttungen ausreicht derart, dass eine Karpulenfüllung mehrere Tage ausreichen kann. Solche Insulinpens erlauben dem Benutzer eine für jede Ausschüttung spezifische Dosis vorzuwählen und entsprechend auszuschütten. Autopens weisen einen motorischen Antrieb auf.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP 1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Verabreichungsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Verabreichungsvorrichtung hin gerichtete Seite oder Richtung.

Unter den Begriffen "Injektionssystem" oder "Injektor" oder "Pen" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird, ggf. nach Ablauf einer Verweildauer während der die medizinische Substanz in das Gewebe diffundieren kann. Eine bestimmte Verweildauer kann beispielsweise auf dem Beipackzettel zum Medikament vorgegeben sein. Somit verbleibt bei einem Injektionssystem oder bei einem Injektor im Unterschied zu einem Infusionssystem die Injektionsnadel nicht über einen längeren Zeitraum von mehreren Stunden im Gewebe.

### FIGUREN

- Fig. 1: zeigt schematisch eine Ausführungsform der erfindungsgemässen Verabreichungsvorrichtung für ein Medikament in einer Geräte- bzw. Systemumgebung
- Fig. 2: zeigt zwei perspektivische Ansichten einer Ausführungsform der erfindungsgemässen Verabreichungsvorrichtung in einem Autopen
- Fig. 3: zeigt die Verabreichungsvorrichtung in einem Autopen in proximaler Ansicht und in zwei Längsschnitten
- Fig. 4: zeigt neun Bedienungsfälle einer Ausführungsform der Verabreichungsvorrichtung
- Fig. 5: zeigt Komponenten einer beispielhaften Systemumgebung einer Ausführungsform der erfindungsgemässen Verabreichungsvorrichtung
- Fig. 6: zeigt beispielhaft einen Benutzungsfall einer geführten Bedienung mit einer erfindungsgemässen Verabreichungsvorrichtung in einer beispielhaften Systemumgebung mit zugehörigen Benutzungsaktionen und Datenflüssen

### FIGURENBESCHREIBUNG

In Zusammenhang mit den angehängten Figuren werden nachfolgend bevorzugte Ausführungsformen bzw. Aspekte der Erfindung beschrieben. Diese sollen grundsätzliche Möglichkeiten und die Leistungsfähigkeit der Erfindung beispielhaft aufzeigen und keinesfalls einschränkend ausgelegt werden.

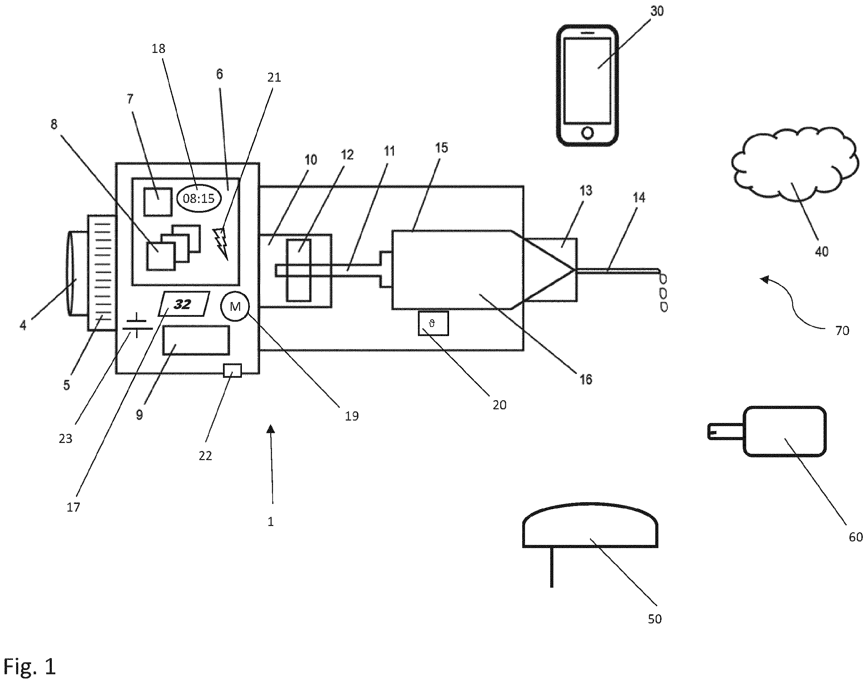

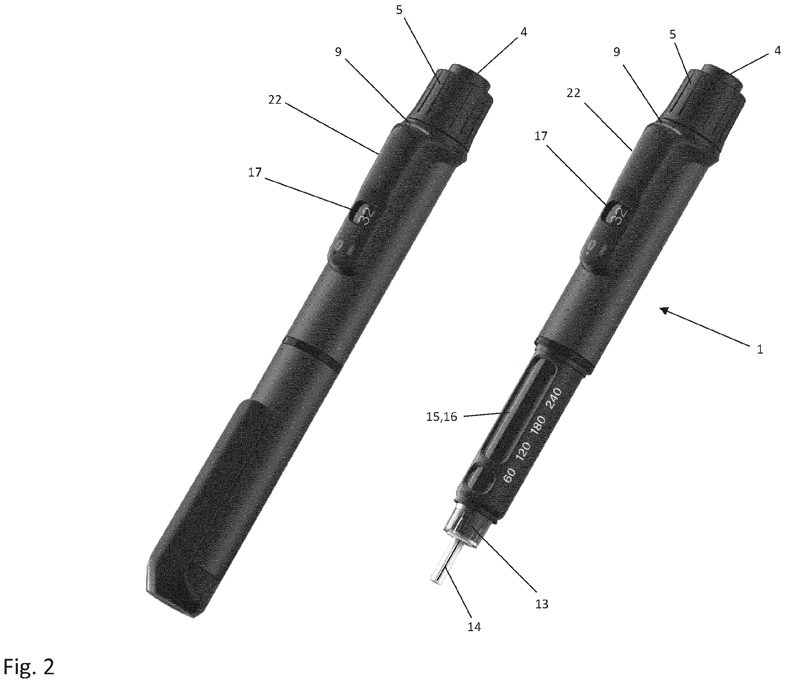

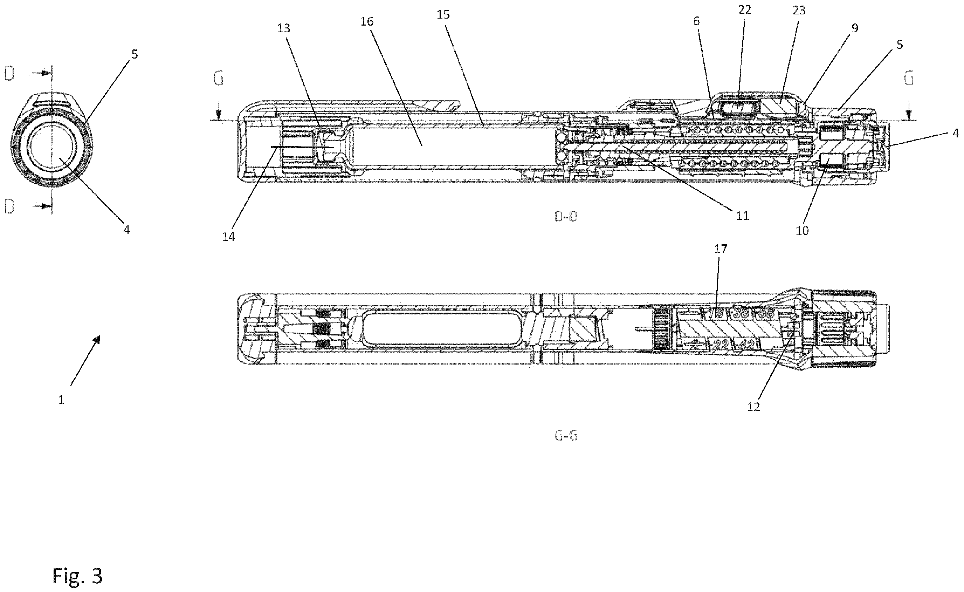

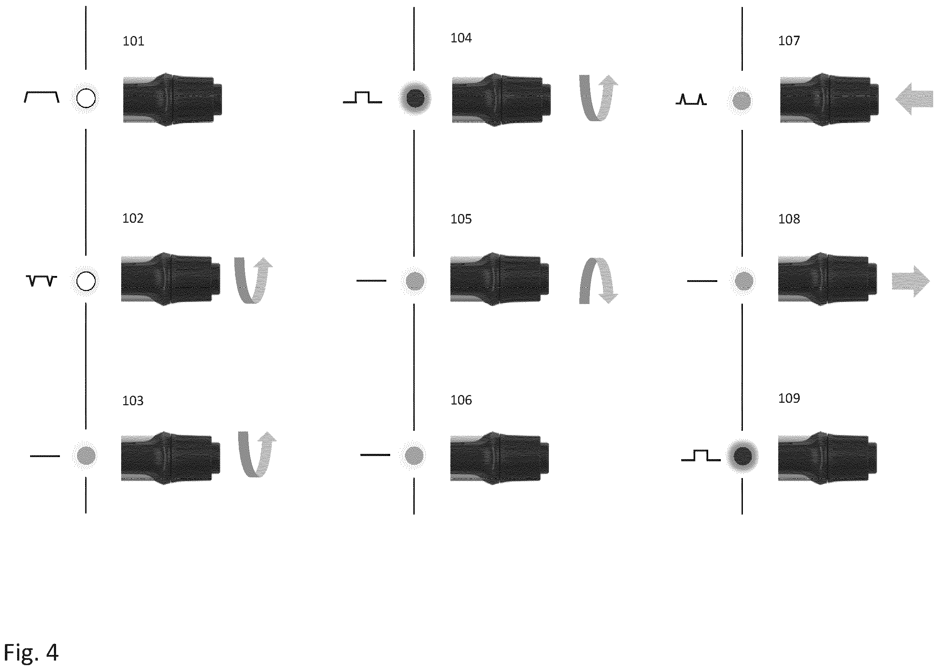

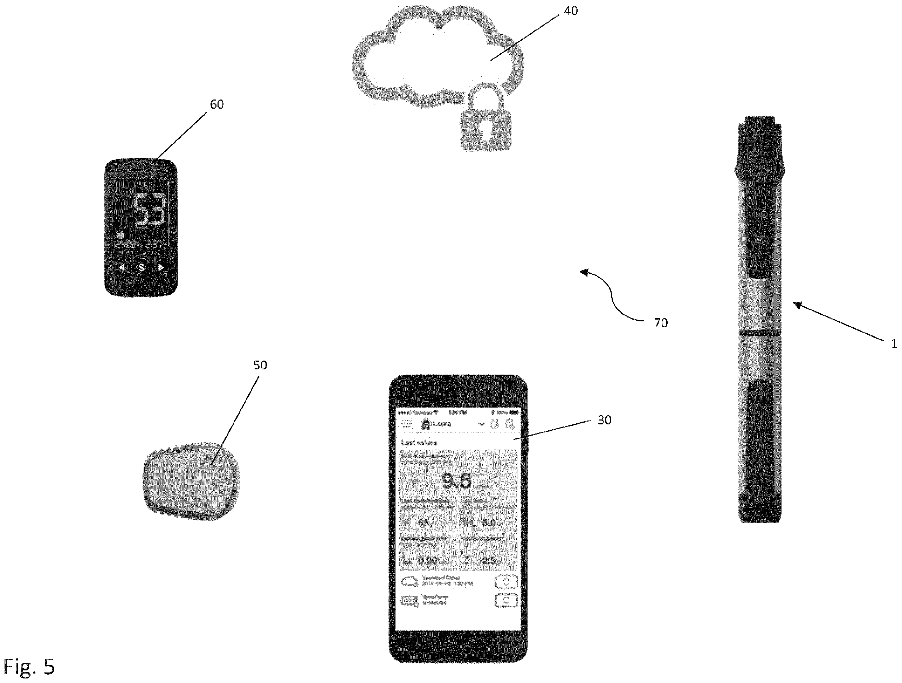

Schematisch ist in Figur 1 eine Ausführungsform der Verabreichungsvorrichtung 1 entsprechend der der Erfindung gezeigt. Ein Vorwahlmittel 5, z.B. in der Form eines Drehrings dient dazu eine auszuschüttende Dosis einzustellen bzw. zu korrigieren. Die aktuell eingestellte Dosis ist dabei auf einer Skala 17 ablesbar, beispielsweise mittels gedruckter Ziffern auf einer Einstellhülse durch ein Sichtfenster in der Verabreichungsvorrichtung 1. Ein Bedienmittel 4, beispielsweise in der Form eines in axialer Richtung betätigbaren Druckknopfs 4 am proximalen Ende der Verabreichungsvorrichtung 1 dient dazu, die eingestellte Dosis auszuschütten. Dazu wird ein Antrieb 10, beispielsweise ein Motor oder ein Schraubgetriebe durch das Bedienmittel 4 eingeschaltet bzw. freigegeben bzw. betätigt. Der Antrieb 10 kann dadurch auf ein Abtriebsmittel 11, beispielsweise eine Kolbenstange mit distal angebrachtem Flansch wirken. Dadurch kann sich für eine Ausschüttung von Medikament 16 das Abtriebsmittel 11 relativ zu einem in der Verabreichungsvorrichtung 1 eingesetzten Behälter 15, beispielsweise einer Karpule mit Stopfen und Septum in distale Richtung axial bewegen, wobei das Medikament 16 mittels der Stopfenbewegung durch eine in das Septum eingestochene Hohlkanüle 14 in der Nadel 13 gepresst wird bis der Antrieb 10 angehalten wird, beispielsweise indem ein Nullanschlag erreicht bzw. die eingestellte Dosis ausgeschüttet ist oder indem das eindrückbare Bedienmittel 4 losgelassen wird. Die Verabreichungsvorrichtung 1 weist eine elektronische Steuerung 6 auf, beispielsweise eine elektronische Baugruppe enthaltend ein Mikroprozessor, einen Speicher 8 für Programme und Daten, eine Zeitmesseinrichtung 7 mit Echtzeituhr 18 und einem Sendeempfänger 21 für drahtlose Kommunikation beispielsweise mittels BLE Technologie. Die Zeitmesseinrichtung 7 ist dazu ausgebildet, dass eine Zeitdauer vom Ende einer Ausschüttung bis zum Loslassen des Bedienmittels 4 laufend gemessen werden kann, wobei der laufende Wert der Zeitdauer ereignis- bzw. programmgesteuert jederzeit aktuell ausgelesen werden kann und der erreichte Wert der Zeitdauer zur Verfügung steht. Insbesondere kann jederzeit aktuell die Relation der Zeitdauer zu einem vorgegeben Grenzwert bestimmt werden um zu ermitteln, ob eine erfolgte Auschüttung als Priming oder als Verabreichung zu charakterisieren ist und/oder die gemessene Zeitdauer kann aktuell oder rückschauend herangezogen werden um zu ermitteln, ob eine Verabreichung betreffend der Verweildauer mit dem bestimmungsgemässen Gebrauch konform ist. Die elektronische Steuerung 6 kann mittels einem oder mehreren Sensoren 12 die rotativen und/oder axialen Bewegungen des Antriebs 10, des Vorwahlmittels 5 sowie des Bedienmittels 4 qualitativ und/oder quantitativ erfassen. Alternativ kann die Betätigung des Bedienmittels 4 auch mittels Berührungssensoren auf beispielsweise kapazitiver, resistiver oder piezoelektrischer Basis oder mittels einem elektromechanischen Schalter festgestellt werden. Die Verabreichungsvorrichtung 1 weist weiter ein Anzeigemittel 9 auf, beispielsweise in Form einer mehrfarbigen (RGB) LED und einem zugehörigen Lichtleiter, dessen Aussenfläche gut sichtbar in der Gehäuseaussenseite der Verabreichungsvorrichtung 1 eingebettet ist. Zusätzlich kann die elektronische Steuerung 6 einen Vibraalarm 19 aktivieren und die Temperatur mittels einem oder mehrerer angeschlossener Temperatursensoren messen und registrieren, beispielsweise kann ein Temperatursensor 20 in der Verabreichungsvorrichtung 1 thermisch mit dem Behälter 15 oder mit dem Medikament 16 verbunden sein. Die elektronische Steuerung 6 wird durch eine in der Verabreichungsvorrichtung eingebaute Energiequelle 23 gespiesen, diese kann beispielsweise aus einem oder mehreren Lithium-Ionen Elementen gebildet sein. Eine Anschluss 22, beispielsweise eine USB-C Buchse dient dazu, die Energiequelle zu laden und/oder Programme und/oder Konfigurationsdaten und/oder Nutzdaten runter- bzw. hochzuladen. Weiter ist in Figur 1 sowie in Figur 5 beispielhaft eine Geräte- bzw. Systemumgebung gezeigt. Diese kann eine Fernsteuerung oder Smartphone 30 aufweisen welches eine oder mehrere Apps enthält, insbesondere zur Führung eines Therapietagebuchs und zur Berechnung von Dosisempfehlungen. Weiter kann die Systemumgebung eines oder mehrere aus Host/PC/Cloudcomputer 40, kontinuierlich arbeitendem Glukosemessgerät 50 und Glukosemessgerät 60 aufweisen. Die Geräte der Systemumgebung sind drahtlos untereinander, insbesondere mit der Verabreichungsvorrichtung 1 verbindbar. Eines oder mehrere der Geräte 30, 40, 50, 60 können als Kontroller 70 für die Verabreichungsvorrichtung 1 fungieren. Im vorliegenden Beispiel übernimmt das Smartphone 30 auch die Kontroller-Funktion als Kontroller 70. Figur 2 zeigt zwei perspektivische Ansichten einer Ausführungsform der erfindungsgemässen Verabreichungsvorrichtung 1 in einem federgetriebenen Autopen mit und ohne Schutzdeckel. Mittels dem Drehring 5 kann die gewünschte Dosis eingestellt werden, wobei gleichzeitig eine Antriebsfeder gespannt wird. Die aktuell eingestellte, bzw. vorgewählte Dosis ist auf der Skala 17 ablesbar. Durch Betätigen des Druckknopfs 4 wird die Verabreichungsvorrichtung 1 aktiviert, wodurch Medikament 16 aus dem Behälter 15 durch die Kanüle 14 in der Nadel 13 ausgeschüttet wird. Die Anzeige 9 vermittelt dem Benutzer / der Patientin Informationen über die aktuell eingestellte Dosis, den Status der laufenden Ausschüttung sowie Informationen zum Betriebszustand der Verabreichungsvorrichtung 1 bzw. der Systemumgebung. Figur 3 zeigt die Verabreichungsvorrichtung 1 bzw. den Autopen in proximaler Ansicht und in zwei Längsschnitten D-D und G-G. Neben den bereits bei Figur 2 beschriebenen Elementen Druckknopf 4, Drehring 5, Karpule 15 mit Medikament 16, Nadel 13 mit Kanüle 14 zeigt die Figur 3 die elektronische Steuerung 6 mit der Anzeige 9, der USB-Buchse 22 und dem Akku 23 den Federmotor 10, die Kolbenstange 11, die bedruckte Skalenhülse 17 sowie eine mit der elektronische Steuerung 6 verbundene Sensorgruppe 12 für rotative und axiale Bewegungen von Teilen der Verabreichungsvorrichtung 1. Die Figur 4 zeigt neun Bedienungsfälle 101 bis 109 einer anhand einer beispielhaften Ausführungsform der Verabreichungsvorrichtung 1 in einem Autopen. Der Fall 101 zeigt die Verabreichungsvorrichtung 1 nach erfolgreichem Einschalten. Ein solcher Einschaltvorgang kann bewirkt werden, indem beispielsweise der Drehring 5 oder der Druckknopf 4 manipuliert wird.

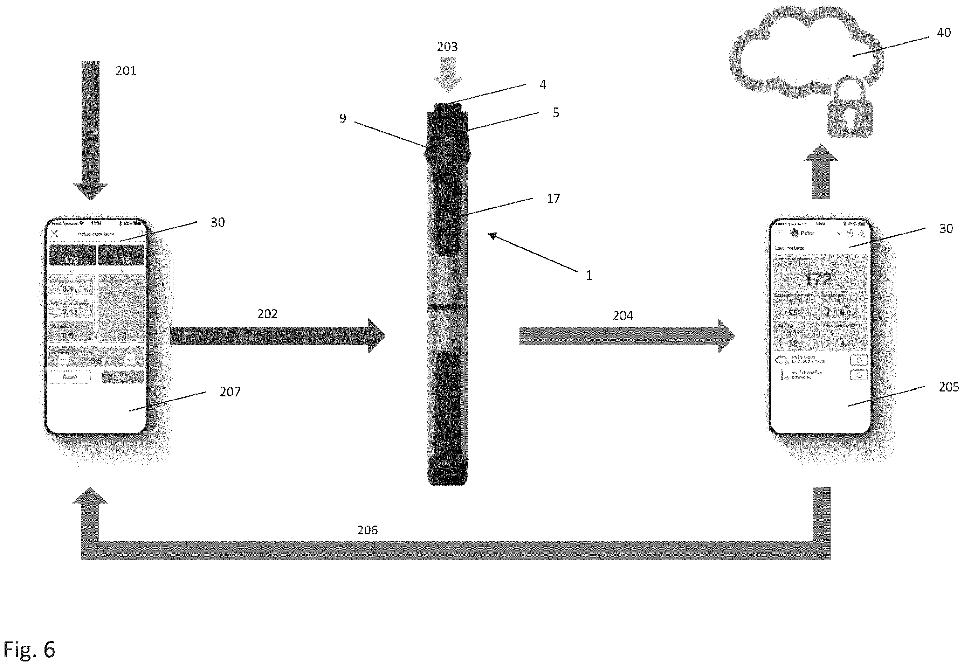

Dadurch wird die elektronische Steuerung 6 eingeschaltet und/oder aus einem Energiesparmodus geweckt, was über die Anzeige 9 signalisiert wird, beispielsweise indem diese dauernd (ununterbrochen) weiss leuchtet. Sobald die Verabreichungsvorrichtung 1 eingeschaltet und mit dem Kontroller 70 verbunden ist kann jene eine Dosisempfehlung bzw. einen Vorgabewert von diesem empfangen. Der Fall 102 zeigt die Verabreichungsvorrichtung 1 beim Aufziehen (Erhöhen) einer Dosis durch Drehen des Drehrings 5 im Uhrzeigersinn. Die Anzeige 9 signalisiert die Rastschritte beim Aufziehen, beispielsweise indem diese das weisse Leuchten pro Dosisschritt bzw. Dosiseinheit kurz unterbricht. Der Fall 103 zeigt die Verabreichungsvorrichtung 1 beim Erreichen der Dosisempfehlung bzw. des Vorgabewerts durch die eingestellte Dosis. Die Anzeige 9 zeigt das Erreichen des Vorgabewerts an und signalisiert dem Benutzer oder der Patientin, dass die empfohlene Dosis erreicht ist, beispielsweise indem die Anzeige 9 in diesem Fall dauernd grün leuchtet. Bei weiterer Erhöhung um einen Rastschritt wechselt die Anzeige auf weiss, um sodann bei weiterer Erhöhung bzw. einer deutlichen Überschreitung des Vorgabewerts wie im Fall 104 gezeigt, beispielsweise nach weiteren zwei Rastschritten oder Dosiseinheiten auf rot zu wechseln. Im Fall 105 wird diese zu hoch vorgewählte Dosis durch Drehen am Drehring 5 im Gegenuhrzeigersinn korrigiert, was durch die Signalisierung mittels der Anzeige 9 geführt bzw. quittiert wird, indem die Anzeige von rot über weiss wieder auf grün wechselt. Dieser Zustand ist in Fall 106 dargestellt, wo die an der Verabreichungsvorrichtung 1 eingestellte Dosis mit dem Vorgabewert übereinstimmt und die Anzeige beispielsweise dauernd grün leuchtet. Der Autopen kann an der Einstichstelle eingestochen werden. Sodann kann wie für den Fall 107 gezeigt durch Eindrücken des Druckknopfs 4 die Ausschüttung veranlasst werden. Die laufende Ausschüttung wir signalisiert, beispielsweise indem die Anzeige grün blinkt. Diese Signalisierung wird nach dem Ende der Ausschüttung während einer vorgegebenen Verweildauer um beispielsweise weitere 10 Sekunden weitergeführt. Wie für den Fall 108 gezeigt wird kann erst nach Ablauf dieser Verweildauer eine Ausschüttung als konforme Verabreichung gewertet werden und dies wird beispielsweise durch grünes Dauerlicht signalisiert, so dass der Druckknopf 4 losgelassen und der Pen von der Einstichstelle entfernt werden kann. Sollten während dem Gebrauch der Verabreichungsvorrichtung 1 Fehler auftreten, werden solche signalisiert wie für den Bedienungsfall 109 gezeigt, beispielsweise durch rotes Aufleuchten oder Blinken der Anzeige 9. Figur 6 zeigt beispielhaft einen Benutzungsfall einer geführten Bedienung mit einer erfindungsgemässen Verabreichungsvorrichtung 1 in einer beispielhaften Systemumgebung mit zugehörigen Benutzungsaktionen und Datenflüssen. Die geführte Bedienung wird anhand einer Verabreichung von Insulin im Rahmen einer Diabetestherapie und anhand von Figur 4 beschrieben. Neben der Verabreichungsvorrichtung 2 mit der Verabreichungsvorrichtung 1 weist die Systemumgebung folgende Komponenten auf: ein Smartphone 30 mit Apps welche ein Diabetestagebuch 205 und einen Bolusrechner 207 implementieren sowie eine Cloud 40 und/oder ein Hostcomputer welcher beispielsweise therapierelevante oder patientenspezifische Daten liefern, verarbeiten oder sichern kann. Der Benutzungsfall kann damit beginnen, dass therapierelevante Eingangsdaten 201 durch den Bolusrechner 207 mittels einem Benutzerinterface auf dem Smartpphone 30 abgefragt werden, beispielsweise als aktueller Glukosewert und als Energiegehalt einer unmittelbar bevorstehenden Mahlzeit. Daraus und aus weiteren Parametern, beispielsweise der Tageszeit oder aus den kürzlich bereits erfolgten Verabreichungen berechnet der Bolusrechner 207 eine Bolusempfehlung 202. Diese kann über einen Dialog mittels Benutzerinterface auf dem Smartphone 30 bestätigt werden und wird sodann als Vorgabewert an die Verabreichungsvorrichtung 1 übertragen. Eine Patientin kann darauf am Drehring 5 geführt über die Anzeige 9 und die Skala 17 die gewünschte Dosis aufziehen und mittels Betätigen des Druckknopfs 4 ausschütten. Wenn nach einer beendeten Ausschüttung die Zeitdauer bzw. die Verweilweildauer der Nadel im Gewebe gleich oder grösser ist als eine vordefinierte Grenzdauer kann die Ausschüttung als therapierelevante Verabreichungsmenge 204 mit Datum- und Zeitstempel an das Diabetestagebuch 205 übermittelt werden. Aus diesem und den vorhergehenden Einträgen im Diabetestagebuch kann dann laufend oder zum Zeitpunkt einer nächsten Bolusberechnung ein "Insulin on board" IOB Wert 206 berechnet werden.

### BEZUGSZEICHENLISTE

- 1: Verabreichungsvorrichtung
- 4: Bedienmittel, Druckknopf
- 5: Vorwahlmittel, Drehring
- 6: elektronische Steuerung
- 7: Zeitmesseinrichtung
- 8: Speicher
- 9: Anzeigemittel
- 10: Antrieb, Federmotor
- 11: Abtriebsmittel, Kolbenstange
- 12: Sensor
- 13: Nadel
- 14: Hohlkanüle
- 15: Behälter, Karpule
- 16: Medikament
- 17: Skala zu Vorwahlmittel
- 18: Echtzeituhr
- 19: Vibraalarm
- 20: Temperatursensor
- 21: Sendeempfänger
- 22: Anschluss, Ladebuchse
- 23: Energiequelle, Akku
- 30: Smartphone, App (s)
- 40: Cloud / Host / PC
- 50: CGM Gerät
- 60: BGM Gerät
- 70: Kontroller (System)
- 101: Gerät eingeschaltet
- 102: Dosis vorwählen
- 103: Vorgabewert bestätigt
- 104: Vorgabewert überschritten
- 105: Vorgabewert bestätigt
- 106: Nadel einstechen
- 107: Ausschüttung und/oder Verweildauer läuft
- 108: Verabreichung beendet
- 109: Fehlersignal
- 201: therapierelevante Eingangsdaten
- 202: Bolusempfehlung bzw. Vorgabewert
- 203: Injektion wird ausgeführt
- 204: Ausschüttmenge mit Zeitstempel und Charakterisierung
- 205: Tagebuch / letzte Verabreichungsmenge
- 206: Insulin on Board (IOB)
- 207: Bolusrechner

## Patentansprüche

1. Verabreichungsvorrichtung (1) zur parenteralen Verabreichung eines Medikaments (16) aufweisend:
- ein Vorwahlmittel (5) mit welchem eine Vorwahlmenge mechanisch oder elektronisch eingestellt und/oder gespeichert werden kann
- einen manuell bewirkbaren Antrieb (10) oder einen freigebbaren motorischen Antrieb (10) welcher ein Abtriebsmittel (11) solange relativ zu einem in die Verabreichungsvorrichtung (1) einsetzbaren Behälter (15) enthaltend ein Medikament (16) bewegt, bis eine der Bewegung des Abtriebsmittels (11) entsprechende, aus dem Behälter (15) durch eine Nadel (13) und/oder Hohlkanüle (14) ausgeschüttete Ausschüttmenge die mit dem Vorwahlmittel (5) eingestellte oder gespeicherte Vorwahlmenge erreicht
- ein manuell betätigbares Bedienmittel (4) welches einen unbetätigten und einen betätigten Zustand aufweisen kann und welches den Antrieb (10) im betätigten Zustand bewirken und/oder freigeben kann
- eine elektronische Steuerung (6) enthaltend eine Zeitmesseinrichtung (7)
**dadurch gekennzeichnet, dass**
die Zeitmesseinrichtung (7) dazu ausgebildet ist, bei betätigtem Bedienmittel (4) eine Zeitdauer von dem Zeitpunkt ab welchem das Abtriebsmittel (11) vom Antrieb (10) nicht mehr bewegt wird bis zum Zeitpunkt in welchem das Bedienmittel (4) in den unbetätigten Zustand übergeht laufend zu messen.

2. Verabreichungsvorrichtung (1) nach Anspruch 1, wobei die elektronische Steuerung (6) dazu ausgebildet ist, die Ausschüttmenge als Primingmenge zu charakterisieren wenn die Zeitdauer kleiner ist als eine vordefinierte Grenzdauer und/oder die Ausschüttmenge als Verabreichungsmenge zu charakterisieren wenn die Zeitdauer gleich oder grösser ist als die vordefinierte Grenzdauer.

3. Verabreichungsvorrichtung (1) nach Anspruch 2, wobei die vordefinierte Grenzdauer als Wert aus einem Bereich von 1 bis 20 sec, bevorzugt 2 bis 5 sec, noch bevorzugter auf 3 sec festgelegt ist oder aus einem Kontroller (70) veränderbar vorgegeben werden kann, wobei der Kontroller (70) eines aus Fernsteuerung, Smartphone App (30), BGM (60), CGM (50), PC/Netzwerk/Cloud/Host/ (40) sein kann oder der Kontroller (70) ein System von mehreren aus Fernsteuerung, Smartphone App (30), BGM (60), CGM (50), PC/Netzwerk/Cloud/Host/ (40) sein kann oder der Kontroller (70) in der Verabreichungsvorrichtung (1) implementiert ist.

4. Verabreichungsvorrichtung (1) nach einem der vorhergehenden Ansprüche, wobei die elektronische Steuerung (6) Bewegung bzw. Abwesenheit von Bewegung des Abtriebsmittels (11) aufgrund eines oder mehrerer Antriebsparameter oder mittels einem Endschalter oder Sensor oder einem rotativen oder linearen Encoder feststellen kann.

5. Verabreichungsvorrichtung (1) nach einem der vorhergehenden Ansprüche, wobei die elektronische Steuerung (6) einen Sensor (12) aufweist, mit welchem die der Bewegung des Abtriebsmittels (11) entsprechende Menge an Medikament (16) quantitativ ermittelt werden kann.

6. Verabreichungsvorrichtung (1) nach einem der Ansprüche 3 bis 5, wobei die elektronische Steuerung (6) eine Echtzeituhr (18) aufweist, insbesondere eine mit einer Uhr im Kontroller (70) synchronisierbare Echtzeituhr (18).

7. Verabreichungsvorrichtung (1) nach einem der Ansprüche 2 bis 6, wobei die elektronische Steuerung (6) einen Speicher (8) aufweist, welcher zumindest die Ausschüttmenge als Verabreichungsmenge aufzeichnet, sofern diese nicht als Primingmenge charakterisiert ist, wobei die Ausschüttmenge oder die Verabreichungsmenge und/oder die Primingmenge je mit einem Zeitstempel und/oder Datumsstempel und/oder zumindest einer weiteren Charakterisierung versehen sein können.

8. Verabreichungsvorrichtung (1) nach einem der Ansprüche 3 bis 7, wobei die elektronische Steuerung (6) einen Sendeempfänger (21) aufweist, womit zu dem Kontroller (70) und/oder zu einem oder mehreren aus Fernsteuerung, Smartphone App (30), BGM (60), CGM (50), PC/Netzwerk/Cloud/Host/ (40) eine drahtlose Verbindung herstellbar ist.

9. Verabreichungsvorrichtung (1) nach dem vorhergehenden Anspruch, wobei die elektronische Steuerung (6) insbesondere über die drahtlose Verbindung vom Kontroller (70) einen Vorgabewert für eine nächste Ausschüttung empfangen kann.

10. Verabreichungsvorrichtung (1) nach dem vorhergehenden Anspruch, wobei die Verabreichungsvorrichtung (1) ein Anzeigemittel (9), insbesondere eine mehrfarbige LED aufweist, welches von der elektronische Steuerung (6) angesteuert signalisieren kann, dass die aktuelle mit der Vorwahlmittel (5) eingestellte Vorwahlmenge zumindest eines aus kleiner, gleich, etwas grösser, viel grösser als der Vorgabewert ist und/oder die Auschüttmenge die mit dem Vorwahlmittel vorgewählte oder die gespeicherte Vorwahlmenge erreicht hat und /oder die aktuelle Zeitdauer zumindest eines aus kleiner, gleich, etwas grösser, viel grösser als die vordefinierte Grenzdauer und/oder eine vorgegebene Verweildauer ist und/oder ein Stopkriterium beispielsweise ein Fehler aufgetreten ist.

11. Verabreichungsvorrichtung (1) nach einem der Ansprüche 3 bis 10, wobei die Verabreichungsvorrichtung (1) eine Skala (17) aufweist, welche die aktuell mit der Vorwahlmittel (5) vorgewählte Menge anzeigen kann oder/und die aktuell mit der Vorwahlmittel (5) vorgewählte Menge durch den Kontroller (70) angezeigt werden kann.

12. Verfahren für die geführte Bedienung einer Verabreichungsvorrichtung (1) zur Verabreichung eines Medikaments (16) aufweisend die Schritte:
- Bereitstellen eines Vorgabewerts und einer vordefinierten Grenzdauer auf einem Kontroller (70)
- Übertragen des Vorgabewerts und der vordefinierten Grenzdauer vom Kontroller (70) an die Verabreichungsvorrichtung (1)
- Einstellen einer Vorwahlmenge an der Verabreichungsvorrichtung (1)
- Signalisieren der Relation Vorwahlmenge zu Vorgabewert durch die Verabreichungsvorrichtung (1), insbesondere signalisieren, dass die Vorwahlmenge identisch zum Vorgabewert ist und/oder, dass die Verabreichungsvorrichtung (1) bereit ist für eine Verabreichung
- Betätigen eines Bedienmittels (4) an der Verabreichungsvorrichtung (1) wodurch Ausschütten von Medikament (16) als Auschüttung erfolgt
- Laufendes Messen einer Zeitdauer ab dem Ende der Ausschüttung bis zur Unbetätigung des Bedienmittels (4)

13. Verfahren nach dem vorhergehenden Anspruch, aufweisend zudem einen oder mehrere der folgenden Schritte:
- Erstellen einer drahtlosen authentifizierten Paarung der Verabreichungsvorrichtung (1) und dem Kontroller (70), insbesondere durch eine "out of band" Methode, beispielsweise mittels QR Code auf der Verabreichungsvorrichtung (1) oder dem darin einsetzbaren Medikamentenbehälter (15)
- Erstellen einer kryptographisch gesicherten drahtlosen Verbindung zwischen der Verabreichungsvorrichtung (1) und dem Kontroller (70)
- Abgleich der gespeicherten Ausschüttmengen, Charakterisierungen, Vorgabewerte, Stati, Parameter, Echtzeituhren, geografischen Koordinaten zwischen der Verabreichungsvorrichtung (1) und dem Kontroller (70)
- Signalisieren, dass die aktuell eingestellte Vorwahlmenge kleiner als der Vorgabewert ist
- Signalisieren, dass die aktuell eingestellte Vorwahlmenge gleich dem Vorgabewert ist
- Signalisieren, dass die aktuell eingestellte Vorwahlmenge grösser als der Vorgabewert ist
- Signalisieren des Endes der Ausschüttung sobald die eingestellte Vorwahlmenge bzw. eine Ausschüttmenge ausgeschüttet ist
- Signalisieren, dass die Zeitdauer die vordefinierte Grenzdauer nicht erreicht
- Signalisieren, dass die Zeitdauer die vordefinierte Grenzdauer erreicht oder überschreitet
- Signalisieren, dass die Zeitdauer eine vorgegebene Verweildauer nicht erreicht
- Signalisieren, dass die Zeitdauer eine vorgegebene Verweildauer erreicht oder überschreitet
- Festhalten der Zeitdauer vom Ende der Ausschüttung bis zur Unbetätigung des Bedienmittels (4) als Haltedauer
- Versehen der Ausschüttmenge mit einem oder mehreren aus Zeitstempel, Medikamentenbezeichnung, Konzentration, Lotnummer, Karpulennummer, Temperatur, geografischer Ort, räumliche Orientierung etc.
- Charakterisieren der Ausschüttmenge als Primingmenge falls die Zeitdauer kleiner als die vordefinierte Grenzdauer ist
- Charakterisieren der Ausschüttmenge als Verabreichungsmenge falls die Zeitdauer grösser oder gleich der vordefinierten Grenzdauer ist
- Charakterisieren der Ausschüttmenge als konforme Verabreichungsmenge falls die Zeitdauer grösser oder gleich der vorgegebenen Verweildauer ist
- Löschen des Vorgabewerts in der Verabreichungsvorrichtung (1) falls die Verabreichungsmenge gleich oder grösser ist als der Vorgabewert
- Speichern der Verabreichungsmenge in der Verabreichungsvorrichtung (1)
- Speichern der Primingmenge in der Verabreichungsvorrichtung (1)
- Übertragen der Verabreichungsmenge von der Verabreichungsvorrichtung (1) an den Kontroller (70)
- Übertragen der Primingmenge von der Verabreichungsvorrichtung (1) an den Kontroller (70)
- Abfragen einer Bestätigung auf dem Kontroller (70) zur Verifikation der letzten Verabreichungsmenge und/oder Primingmenge

14. Verfahren nach dem vorhergehenden Anspruch, aufweisend zudem einen oder mehrere der folgenden Schritte:
- Vermindern des Vorgabewerts um die Verabreichungsmenge in der Verabreichungsvorrichtung (1)
- Übertragen des verminderten Vorgabewerts von der Verabreichungsvorrichtung (1) an den Kontroller (70)
- Aufforderung auf dem Kontroller (70) zum Wechsel des Behälters (15) in der Verabreichungsvorrichtung (1)
- Aufforderung auf dem Kontroller (70) zum Durchführen von Priming an der Verabreichungsvorrichtung (1)

15. Verfahren nach einem der Ansprüche 12 bis 14 ausgeführt auf einer Verabreichungsvorrichtung (1) nach einem der Ansprüche 1 bis 11
